# EUROPEAN PATENT APPLICATION

(11) **EP 2 468 235 A1**
(43) Date of publication of application: **27.06.2012**
(21) Application number: 10196875.8
(22) Date of filing: 23.12.2010
(51) Int. Cl.: A61K 8/02, A61Q 19/10

(54) **Cleaning implement**

(71) Applicant: Unilever PLC, A Company Registered in England and Wales under Company no. 41424, London EC4Y 0DY Greater London (GB); Unilever NV, 3013 AL Rotterdam (NL)
(72) Inventor: Barnwell, Stephen George, Wirral, Meerseyside CH63 3JW (GB); Crawford, Robert John, Wirral, Meerseyside CH63 3JW (GB); Hague, Jonathan David, Wirral, Meerseyside CH63 3JW (GB); Unali, Giovanni Francesco, Wirral, Meerseyside CH63 3JW (GB)
(74) Representative: Mulder, Cornelis Willem Reinier

(57) **Abstract**

A dry cleansing implement comprising adjacent layers of compressed sponge, the layers having sandwiched therebetween a cleansing composition.

## Description

The present invention relates to a cleaning implement, for example conveniently used for personal care cleansing or hard surface cleaning utilities.

The advantages of disposable single-use cleaning implements are known and understood. They may be more sanitary, and if impregnated with a composition they are designed to deliver to a surface to be cleaned, they may provide an extra degree of convenience in that their use obviates the need to apply the substance to the implement just prior to application. As such, with the use of such products, it may no longer be necessary for the user to have or carry a container of the composition to be applied.

There are a variety of impregnated disposable single-use cleaning implements in the patent literature. For example, EP-A-1,459,672 discloses a cleaning implement including a first outer layer, a second outer layer, and an inner layer disposed between the outer layers. The inner layer retained between the outer layers may expand upon contact with water, such that the final height of the implement may be greater than about 1.5 times the initial height of the implement. A suggested material for the inner layer is a sponge, for example made of cellulose derivatives.

WO-A-01/08641 describes dry, disposable personal care articles comprising a first layer comprising a non-woven ply and a polymeric net arranged with the non-woven ply, and a cleansing component and/or therapeutic benefit component arranged adjacent to the first layer. The article may optionally comprise a second layer, which comprises materials selected from non-wovens, wovens, sponges (e.g. natural and synthetic), formed films, battings, and combinations thereof. The second layer is said to be useful for providing a different textured side to the article, as well as acting as an addition carrier for the cleansing component. A preferred second layer comprises batting which comprises synthetic fibres.

WO-A-99/13861 describes substantially dry, disposable, personal cleansing articles having a substrate which comprises multiple layers, the article having a water insoluble substrate having at least a first portion that is wet-extensible, a second portion that is less wet-extensible than the first portion, and a lathering surfactant added onto or impregnated into the substrate.

WO-A-01/08655 describes cleansing articles comprising a water-insoluble substrate comprising a soft first layer and an exfoliating second layer disposed adjacent to the first layer.

WO 2009/040428 describes a cleansing implement comprising a generally planar compressed water-swellable matrix and a solid cleansing composition coated on one face of the compressed matrix.

There remains however the need for a cleaning implement which provides good delivery of surfactant and/or any other ingredients to be applied, such as for example skin care actives, moisturisers, or indeed other agents conventionally used in skin cleansing compositions, or other compositions intended for cleaning and protecting hard surfaces in homes, restaurants, hospitals and the like. There is also a need for such cleaning implements which occupy less volume, on the basis that if they are used as a single-use disposable implement, the ability to buy packs containing large numbers of them is compromised if they have a large volume. There is also a need for implements which can have improved dosing of the surfactant and other components to the implement. There is also a need for such implements which provide an interesting consumer experience in use. There is also a need for a cleaning implement capable of delivering more than one payload.

Thus, according to a first aspect of the invention, there is provided a dry cleansing implement comprising adjacent layers of compressed sponge, the layers having sandwiched therebetween a cleansing composition.

Preferably, the sponge is reticulated.

Conveniently, the implement is suitable for use on human skin. In other embodiments, it may be suitable for the cleaning, sanitising or disinfecting of hard surfaces.

Conveniently, the implement expands on contact with water.

The implement is substantially dry - that is, the implement (including any surfactant composition and any other components designed to leach out of the implement in use) comprising less than 15 wt%, preferably less than 10 wt.%, preferably less than 5 wt.%, and in some embodiments preferably less than 2 wt.% water. Accordingly, the composition contained within the implement conveniently comprises less than 10 wt.%, preferably less than 5 wt.%, and in some embodiments preferably less than 2 wt.% water.

Conveniently, the implement is compressed such that it has an expanded average depth A, but in it compressed form has an average depth of 0.01 to 0.5A, preferably 0.05 to 0.2A. In some embodiments, it may have a compressed depth of 0.08A to 0.12A.

In an embodiment, the cleansing composition in the implement is preferably in solid form. In some embodiments, the cleaning composition may be located adjacent layers of compressed sponge in a sheet form. In such embodiments, the solid cleansing composition may be in the form of a powder or a pre-formed sheet, which can advantageously be retained between the layers of the compressed sponge, thereby preventing leaching of the cleansing composition out of the implement too quickly.

An advantage of such embodiments is that the rate of leaching of the surfactant and/or other components retained between the layers of compressed sponge may be sufficiently slow and controlled as to make the implement suitable for use on more than one occasion, e.g. up to ten occasions, typically between two and six occasions.

In some embodiments, the cleaning composition may additionally be impregnated into one or both layers of compressed sponge.

The implement according to the invention is preferably peripherally sealed. By this is meant that the adjacent layers of compressed sponge are sealed around their peripheral edges, or close to the peripheral edge (e.g. within a distance of about 10% of the longest dimension of the implement from the peripheral edge). Bonding of the adjacent layers of compressed sponge to each other can be achieved in any conventional manner, for example by chemical bonding, thermal bonding or mechanical bonding.

The implement of the invention conveniently has one or more pockets located between the adjacent layers of compressed sponge, at least one of which contains the cleansing composition. Conveniently however this pocket or pockets can be used to contain other compositions or ingredients which are beneficially delivered from the implement, which compositions or ingredients will be dictated by the intended and use. The use of more than one pocket may for example be beneficial in instances where there is an incompatibility between materials to be contained and dispensed - in any event, different pockets can be used to contain different materials. The exact position of pockets, and any sealing lines in the implement, can selected so as to provide an ergonomic shape to the implement (especially when expanded), or for other functional or aesthetic reasons.

For example, the treatment composition for the article to be used for personal cleansing can include, in addition to surfactants and preferably lathering surfactants, emollients, moisturisers, lubricants, protectants, deodorants or medicaments. The compositions or ingredients can be distributed in different pockets, if the implement has more than one pocket.

The treatment composition for the implement when it is to be used for cleaning, sanitizing and disinfecting hard surfaces, can include, in addition to surfactants, an antiseptic, antibacterial, wax, waterproofing, polishing or other agents as are conventionally used in cleaning compositions intended for cleaning and protecting hard surfaces in homes, restaurants, hospitals, nursing homes for private and industrial use and the like. The implements can be used as mopping and dusting cloths, and as polishing and cleaning cloths.

In a preferred embodiment for personal skin care, the treatment composition includes a surfactant and at least one member of the group of emollients, moisturisers, lubricants, conditioning agents, protectants, deodorants and medicaments. When the implement is exposed to water at the point of use and pressure applied, as by squeezing, an unstable emulsion and in the case of the use of a foaming surfactant, a lather is formed which releases the components of the cleansing treatment composition onto the skin or hair of the individual.

The treatment composition for cleansing, sanitizing and disinfecting hard surfaces and for cleansing and conditioning the skin can further include a visual indicator, such as a water soluble colorant or dye, whose disappearance signifies the depletion of the treatment composition upon repeated application. The water soluble colorant or dye can be independently dissolved or dispersed in the treatment composition and is released gradually during use and depleted at about the same time the active ingredients in the treatment composition are exhausted.

An essential element of cleansing compositions used according to the present invention is that of a lathering surfactant. By a "lathering surfactant" is meant a surfactant, which when combined with water and mechanically agitated generates a foam or lather. Preferably, these lathering surfactants should be mild, which means that they must provide sufficient cleansing or detersive benefits but not overly dry the skin or hair, and yet meet the lathering criteria described above.

The implement products of the present invention typically include at least one lathering surfactant in an amount from about 0.1 % to about 95%, preferably from about 0.75% to about 40%, and more preferably from about 1 % to about 20%, based on the weight of the composition contained in the implement. The total of lathering surfactants will typically be present in amount from about 0.1% to about 95%, preferably from about 5% to about 70%, optimally from about 10% to about 50% by weight of the cleansing compositions.

Conveniently, at least about 10% by weight, preferably at least 20% by weight, preferably at least about 30%, 40%, 50%, 70% or 90% by weight of the total surfactant species used in the implement are anionic surfactants.

A wide variety of lathering surfactants are useful herein and include those selected from the group consisting of anionic, nonionic, cationic, amphoteric and lathering surfactant mixtures thereof.

A preferred anionic surfactant for use in the implements according to the invention is soap. Soap is typically a salt, such as a sodium or potassium salt, of an animal or vegetable fat. Soaps tend to have carbon chain lengths in the region of C₈ to C₂₂, more often C₁₀ to C₁₈.

Among the synthetic anionic lathering surfactants useful herein are the following non-limiting examples which include the classes of:
(1) Alkyl benzene sulfonates in which the alkyl group contains from 9 to 15 carbon atoms, preferably 11 to 14 carbon atoms in straight chain or branched chain configuration. Especially preferred is a linear alkyl benzene sulfonate containing about 12 carbon atoms in the alkyl chain.
(2) Alkyl sulfates obtained by sulfating an alcohol having 8 to 22 carbon atoms, preferably 12 to 16 carbon atoms. The alkyl sulfates have the formula ROSO₃⁻M⁺ where R is the C₈₋₂₂ alkyl group and M is a mono- and/or divalent cation.
(3) Paraffin sulfonates having 8 to 22 carbon atoms, preferably 12 to 16 carbon atoms, in the alkyl moiety. These surfactants are commercially available as Hostapur SAS from Hoechst Celanese.
(4) Olefin sulfonates having 8 to 22 carbon atoms, preferably 12 to 16 carbon atoms. Most preferred is sodium C₁₄-C₁₆ olefin sulfonate, available as Bioterge AS 40®.
(5) Alkyl ether sulfates derived from an alcohol having 8 to 22 carbon atoms, preferably 12 to 16 carbon atoms, ethoxylated with less than 30, preferably less than 12, moles of ethylene oxide. Most preferred is sodium lauryl ether sulfate formed from 2 moles average ethoxylation, commercially available as Standopol ES-2®.
(6) Alkyl glyceryl ether sulfonates having 8 to 22 carbon atoms, preferably 12 to 16 carbon atoms, in the alkyl moiety.
(7) Fatty acid ester sulfonates of the formula: R¹CH(SO₃ ⁻M+)CO₂R² where R¹ is straight or branched alkyl from about C₈- to C₁₈, preferably C₁₂ to C₁₆, and R² is straight or branched alkyl from about C₁ to C₆, preferably primarily C₁, and M+ represents a mono- or divalent cation.
(8) Secondary alcohol sulfates having 6 to 18, preferably 8 to 16 carbon atoms.
(9) Fatty acyl isethionates having from 10 to 22 carbon atoms, with sodium cocoyl isethionate being preferred.
(10) Dialkyl sulfosuccinates wherein the alkyl groups range from 3 to 20 carbon atoms each.
(11) Alkanoyl sarcosinates corresponding to the formula RCON(CH₃)CH₂CH₂CO₂M wherein R is alkyl or alkenyl of about 10 to about 20 carbon atoms and M is a water-soluble cation such as ammonium, sodium, potassium and trialkanolammonium. Most preferred is sodium lauroyl sarcosinate.
(12) Alkyl lactylates wherein the alkyl groups range from 8 to 12 carbon atoms, with sodium lauroyl lactylate sold as Pationic 138C® available from the Patterson Chemical Company as the most preferred.
(13) Taurates having from 8 to 16 carbon atoms, with cocoyl methyl taurate being preferred.

Nonionic lathering surfactants suitable for use in the present invention include C₁₀-C₂₀ fatty alcohol or acid hydrophobes condensed with from 2 to 100 moles of ethylene oxide or propylene oxide per mole of hydrophobe; C₂-C₁₀ alkyl phenols condensed with from 2 to 20 moles of alkylene oxides; mono- and di-fatty acid esters of ethylene glycol such as ethylene glycol distearate; fatty acid monoglycerides; sorbitan mono- and di-C₈-C₂₀ fatty acids; and polyoxyethylene sorbitan available as Polysorbate 80 and Tween 80® as well as combinations of any of the above surfactants.

Other useful nonionic surfactants include alkyl polyglucosides, saccharide fatty amides (e.g. methyl gluconamides) as well as long chain tertiary amine oxides. Examples of the latter category are: dimethyldodecylamine oxide, oleyldi(2-hydroxyethyl)amine oxide, dimethyloctylamine oxide, dimethyldecylamine oxide, dimethyltetradecylamine oxide, di(2-hydroxyethyl)tetradecylamine oxide, 3-didodecyloxy-2-hydroxypropyidi(3-hydroxypropyl)amine oxide, and dimethylhexadecylamine oxide.

Amphoteric lathering surfactants useful for the present invention include aliphatic secondary and tertiary amines, preferably wherein the nitrogen is in a cationic state, in which the aliphatic radicals can be straight or branched chain and wherein one of the radicals contains an ionizable water solubilizing group such as carboxy, sulphonate, sulphate, phosphate or phosphonate. Illustrative substances are cocamidopropyl betaine, cocamphoacetate, cocamphodiacetate, cocamphopropionate, cocamphodipropionate, cocamidopropyl hydroxysultaine, cetyl dimethyl betaine, cocamidopropyl PG-dimonium chloride phosphate, coco dimethyl carboxymethyl betaine, cetyl dimethyl betaine and combinations thereof.

The outer layers of the article of the invention may have different textures and abrasiveness. Differently textured surfaces can be adapted so as to provide an abrasive side for more intensive cleaning and a softer absorbent side for lighter and more gentle cleaning.

The implements of the present invention have a number of advantageous embodiments and aspects.

For example, an advantage of the present invention, where the surfactant is retained in a pocket between adjacent layers of adjacent sponge material, is that the surfactant is more readily retained in the implement both prior to and during use, compared to implements in which the surfactant is simply applied, for example as a sheet to an exterior face of the implement. The benefits of this are that the surfactant is not lost prior to use, and that the implement may be suitable for multiple usages.

In a preferred embodiment, in addition to the implement containing non-encapsulated surfactant, the implement may additionally and beneficially comprise an encapsulated component located in one or more capsules between the layers of the implement, for example an encapsulated perfume, or an emollient, lubricant or moisturiser. The encapsulated ingredient may conveniently be released in a sequential manner relative to the cleansing composition. That is, the user may conveniently take a new implement, wet it and generate a lather which the user then uses to wash themselves or a hard surface. However, thereafter the user may then squeeze the implement harder in order to release the encapsulated ingredient from one or more capsules in the pocket of the implement, thereby providing an implement and process which provides not only a washing or cleansing step, but a subsequent dispensing step in which an encapsulated component is subsequently dispensed.

In an envisaged embodiment, the implement may therefore have a sequential dual function - the implement may for example initially be wetted and used as a cleansing implement, but thereafter an encapsulated component (such as but not limited to a perfume) can thereafter be released, allowing the implement to have a subsequent air freshening utility.

Although specifically disclosed in the context of a cleansing implement/air freshener duality of intended utilities, the skilled person will readily be aware of other pairs of utilities the disclosed implement can be contemplated for, via a sequential dispensing of a second initially encapsulated active material.

In a facet of such sequential dispensing by using an encapsulated material, the term "capsule" is to be interpreted broadly, and could include for example sachets, ampules or vials. Additionally, any capsule use in the invention could be frangible, i.e. it readily snaps and breaks or it could be water-soluble, as the context requires. Either type of capsule would allow for the sequential release of the encapsulated material envisaged according to the invention.

Conversely, such an implement containing an encapsulated component can advantageously be utilised in a reverse sequence. For example, such an implement could advantageously contain an encapsulated disinfectant material, which disinfectant material would be one suitable for either hard surfaces or topical applications. In use, the user can initially squeeze the frangible capsule or capsules to release the disinfectant material, which may be useful for e.g. treating acne on a user's face. Thereafter, the user can wet the implement to cause the surfactant component to be released, and then wash with the implement in a normal manner.

Such a method and implement involving encapsulated materials may also be useful where there is a chemical incompatibility between the surfactant employed and the encapsulated material. In such instances it would be beneficial to provide an implement having two or more pockets, with substances that are incompatible being housed in different pockets.

In a further embodiment, the implement may include a component in between the layers of the implement which is capable of providing an audible feedback to the user that the implement has been squeezed sufficiently, for example to release the surfactant, or to signal to the user that an encapsulated material included between the layers has been released. An example of such a component is "Bubble Wrap" (trade mark).

The invention will now be further described by way of example only.

### Example 1

### Preparation of implement designed for multiple shower / bath uses

Two compressed cellulose sponges each already coated with surfactant sheet were put face-to-face with additional sheets of loose soap paper contained between the two sponge layers. These soap sheets were included in the centre of the sponge by sewing around the perimeter. Once immersed in water they swelled, and crucially, continued to lather sufficiently long to allow for multiple showers.

### Example 2

### Preparation of swells designed to deliver an additional active from a poppable capsule, other than detergent, before during or after the surfactant formulation has been exhausted

Examples of poppable capsules were prepared by aspirating a suitable amount of fluid active to be encapsulated (e.g. topical skin formulation, ointment, perfume, disinfectant etc) into a plastic pipette, taking care not to include too much air. Since air is compressible, it makes the popping of the capsule more difficult. The pipette is inverted, and the end is subsequently sealed (in this case heat sealed) and separated from the rest of the pipette which serves no encapsulating function. The capsule is placed in the pocket generated when two layers of sponge are sewn together, and sealed within. The capsule is popped either before, during or after the use of the surfactant formulation.

### Example 3

### Sandwiched Implements

Sandwiched "double implements" have been prepared by peripherally sewing, stapling and or gluing together of two compressed sponges; when gluing was used as the preferred joining method, care was taken to apply the glue to the cellulose sponges and not the surfactant sheet, to promote the formation of a durable implement.

## Claims

1. A dry cleansing implement comprising adjacent layers of compressed sponge, the layers having sandwiched therebetween a cleansing composition.

2. A dry cleansing implement according to claim 1, wherein the sponge is reticulated.

3. A dry cleansing implement according to claim 1 or 2, wherein the implement expands on contact with water.

4. A dry cleansing implement according any preceding claims, wherein the implement is peripherally sealed.

5. A dry cleansing implement according to any preceding claims, wherein the implement comprises a pocket which contains an additional material comprising an emollient, moisturiser, lubricant, protectant, deodorant or medicament.

6. A dry cleansing implement according to claim 5, wherein the additional material is encapsulated.

7. A dry cleansing implement according to any preceding claims, wherein the cleansing composition comprises a lathering surfactant.

8. A dry cleansing implement according to any preceding claims, wherein the cleansing composition comprises soap.

9. A method of cleansing human skin or cleaning a hard surface comprising:
a. providing a dry implement comprising adjacent layers of compressed sponge, the layers having sandwiched therebetween a cleansing composition and an encapsulated material;
b. wetting the implement to release the cleansing composition; and
c. thereafter squeezing the implement to release the encapsulated material.

10. A method of cleansing human skin or cleaning a hard surface comprising:
a. providing a dray implement comprising adjacent layers of compressed sponge, the layers having sandwiched therebetween a cleansing composition and an encapsulated material:
b. squeezing the implement to release the encapsulated material; and
c. thereafter wetting the implement to release the cleansing composition.

11. A method according to claim 9 or claim 10, wherein the implement can be used for a second utility in addition to cleansing, the second utility being dictated by the encapsulated material.

12. A method according to claim 11 wherein the encapsulated material; is a perfume, preferably wherein the second intended use is as an air freshener.
